# EUROPEAN PATENT SPECIFICATION

(11) **EP 1 829 132 B1**
(45) Date of publication and mention of the grant of the patent: **06.10.2010**
(21) Application number: 04805031.4
(22) Date of filing: 23.12.2004
(51) Int. Cl.: H01L 51/30, G09G 3/32, C07C 255/42

(54) **MATERIAL FOR DOPED AND UNDOPED HOLE AND ELECTRON TRANSPORT LAYER**
MATERIAL FÜR EINE DOTIERTE UND UNDOTIERTE LOCH- UND ELEKTRONENTRANSPORTSCHICHT
MATERIAU POUR COUCHE DE TRANSPORT DE TROUS ET D'ELECTRONS DOPES ET NON DOPES

(43) Date of publication of application: 05.09.2007
(73) Proprietor: Technische Universität Braunschweig, 38106 Braunschweig (DE); BASF SE, 67056 Ludwigshafen (DE)
(72) Inventor: JOHANNES, Hans-Hermann, 38124 Braunschweig (DE); KOWALSKY, Wolfgang, 38116 Braunschweig (DE); DOBREVA, Guergana, 38106 Braunschweig (DE); DÜMELAND, Martin, 06114 Halle (DE); LAWRENTZ, Ulf, 67065 Ludwigshafen (DE)
(74) Representative: Taruttis, Stefan Georg
(86) International application number: PCT/EP2004/053704
(87) International publication number: WO 2006/066630

(56) References cited:
- US-A- 5 286 589
- US-A1- 2004 131 885
- X. ZHOU ET AL: "Very-low-operating-voltage organic light-emitting diodes using a p-doped amorphous hole injection layer" APPLIED PHYSICS LETTERS, vol. 78, no. 4, 22 January 2001 (2001-01-22), pages 410-412, XP012028438 cited in the application
- BECKER, JAMES Y ET AL: "Synthesis and Properties of Archetypal Donor-Acceptor-Donor (D2A) Molecules" J. ORG. CHEM., vol. 53, no. 8, 1988, pages 1689-1694, XP002347723

## Description

The present invention relates to materials useful as a hole or electron transport layer or as a dopant for hole or electron transport layers as well as for hole or electron injection layers in organic electric, preferably electrooptic devices. The material usable as a hole or electron transport layer (HTL and ETL, respectively) or a hole or electron injection layer can form or be part of an electrically conductive organic layer, suitable for the transport of so-called positive charges or holes. Electrooptic devices for the purposes of this disclosure comprise organic light emitting diodes (OLEDs), organic field effect transistors (OFETs), lasers and photovoltaic devices suitable for photovoltaic solar energy conversion.

### State of the art

In general, electrooptic devices comprise a plurality of electrically conductive organic compounds which are stacked in layers which are arranged between electrodes.

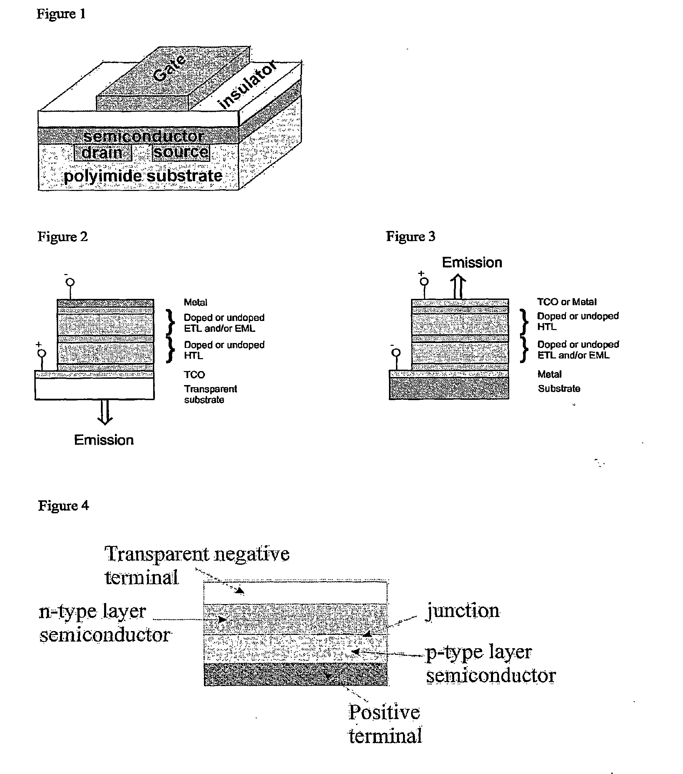

The general structure of EL devices, which the inventive material for a hole transport layer can be utilized is depicted schematically in Figures 1 to 4.

In the case of OLBDs, adjacent the anode, consisting for example of ITO (indium tin oxide), there is a hole transport layer (HTL), optionally with an intermediate hole injection layer (HII). Next to the HTL, an emissive layer is arranged, the compounds of which generally emit visible light, with the energy stemming from an exciton generated by the simultaneous localization of an electron and a hole on the same molecule within the emissive layer, transport layer and adjacent the emissive layer, there is arranged an electron transfer layer and, subsequently, a cathode (for example Mag, LiF/Al, Ca, Ba).

Optionally, electron transport beyond the emissive layer towards the anode may be prevented by an electron blocking layer arranged between the hole transport layer and the emissive layer. As a further option, the migration of holes beyond the emissive layer towards the cathode may be prevented by a hole blocking layer arranged between the emissive layer and the electron transfer layer.

There may be arranged an electron injection layer between the electron transfer layer and the cathode. Further, the electron transfer layer or electron injection layer may be separated from the cathode by an electron conductive protective layer in order to allow the processing steps necessary for applying the cathode onto the electron injection layer.

Materials for hole transport layers (HTL) are known from WO 20041016711 A1, for example α-NPD as an intrinsic HTL or m-MTDATA, p-doped with F₄-TCNQ.

US 5,286,589 describes photosensitive compounds for coating a photosensitive drum of a photocopier. These compounds are described as generating charge upon irradiation, i.e. as charge generating materials of a photosensitive layer. Separate from these charge generating compounds, 2,4,7- trinitrofluorenone, 2,4,5,7- tetranitrofluorenone, chloranil, tetracyanoquinodimethane, pyrene, anthracene, carbazole, indole, imidazole, oxazole, thiazole, oxadiazole, pyrazoline, thiadiazole, triazole, and the hydrazone compounds p-diethylaminobenzaldehyde-*N,N*-diphenylhydrazone, *N*,*N*-diphenylhydrazino-3-methylidene-9-ethylcarbazole, and the styryl compounds α-phenyl-4,4'-*N,N*-diphenylaminostylbene, 5-[4-(di-p-tolylamino)benzylidene]5*H*-dibenzo[*a*,*d*]cycloheptene, and generally benzidine compounds, triarylmethane compounds, triphenylamines, poly-*N-*vinylcarbazole, and polyvinylanthracene are mentioned as charge transporting compounds.

Zhou et al. (Applied Physics Letters, Vol. 78, No. 4, pages 410 to 412) disclose OLEDs using a p-doped amorphous hole injection layer. A hole transport layer of both polycrystalline phthalocyanines and amorphous 4, 4', 4' tris-(*N*,*N*- diphenylamine)triphenylamine (TDATA) p-doped by co-evaporation with F₄-TCNQ (tetrafluoro-tetracyano-quinodimethane) was shown to yield a conductivity orders of magnitude above that of undoped matrix materials. Zhou et al. show that p-doping of the matrix material leads to a larger current density at lower voltages applied as well as to maximum electroluminescence (EL) efficiencies at lower driving voltages.

As a result, OLEDs containing p-doped HTLs exhibit a very low operating voltage and an improved EL efficiency as a result of controlled doping.

### Objects of the invention

It is an object of the present invention to provide an alternative to p-doped HTL materials for use in organic electrooptic devices.

In its preferred embodiment, the present invention seeks to provide HTL materials suitable for organic electrooptic devices that have improved characteristics, like an increased stability at elevated temperatures.

It is a further object of the invention to provide a method for synthesis of novel HTL materials.

### General description of the invention

The present invention achieves the above-mentioned objects by providing a material suitable for a hole and electron transport and/or injection layer in organic electrooptic devices according to the following general formula I:

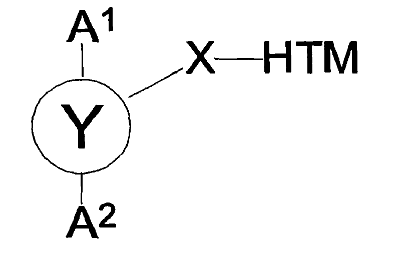

In general formula I, moiety Y represents a central carbon based structure, optionally comprising hetero atoms, for example having 5 to 14 atoms, conjugatedly linking accessory residues A¹ and A². Residues A¹ and A² are electron accepting residues, having at least one π-bond, and are conjugated through moiety Y to form a conjugated or aromatic system. In addition to accessory residues A¹ and A², further electron donating groups may be conjugatedly linked to moiety Y. In addition to residues A¹ and A², a hole transport moiety (HTM) or more HTMs are covalently linked to moiety Y by intermediate moiety X. The at least one HTM is capable of hole transporting electric charges by the mechanism known as hole transport. However, the at least one HTM is not conjugated linked to moiety Y. Intermediate moiety X can be a chemical bond or any carbon atom and/or heteroatom comprising moiety suitable to non-conjugatedly link the at least one HTM to Y.

The number of HTMs ranges from at least one to a maximum that moiety Y is capable of non-conjugatedly linking. As an example for moiety Y being a five- or six-membered carbon ring, optionally substituted with heteroatoms, the number of HTMs can be 1, 2, 3 or 4. The number of residues X and of HTM can increase when moiety Y consists of one, two or more condensed rings, e.g. comprising a total of 5 to 14 carbon atoms or heteroatoms.

Examples for moiety Y are shown in the following embodiments of general formula I, wherein the designations refer to moiety Y derivatized with Xs, A¹ and A²:

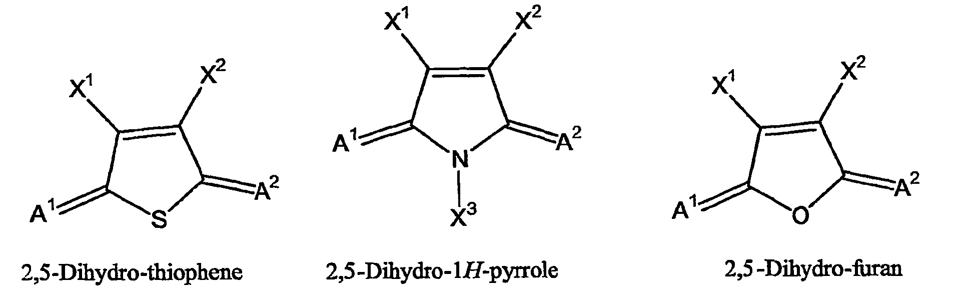

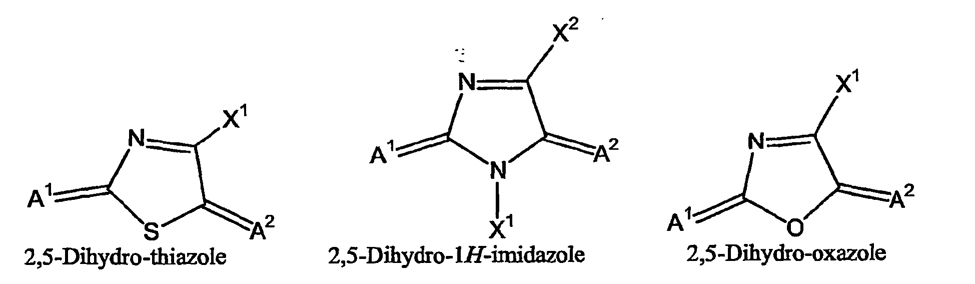

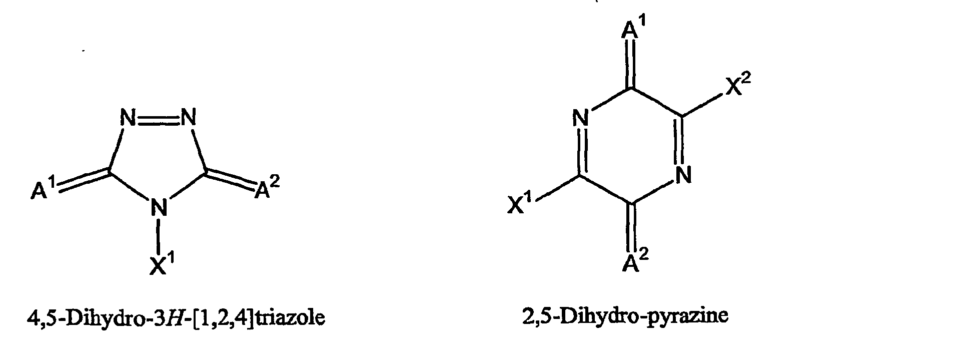

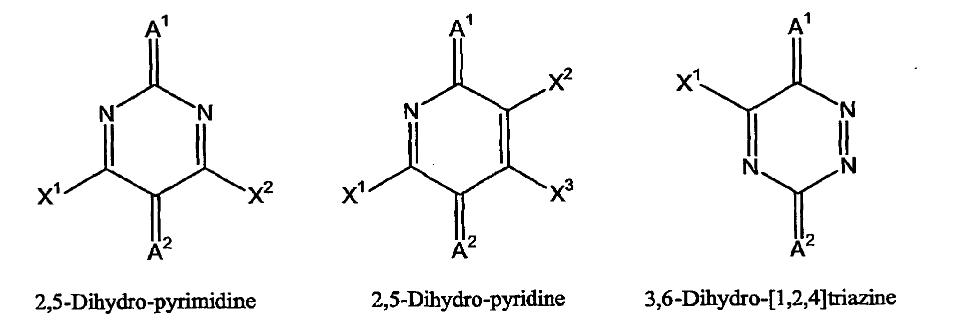

Further embodiments of general formula I are represented by compounds of formulae II to VI:

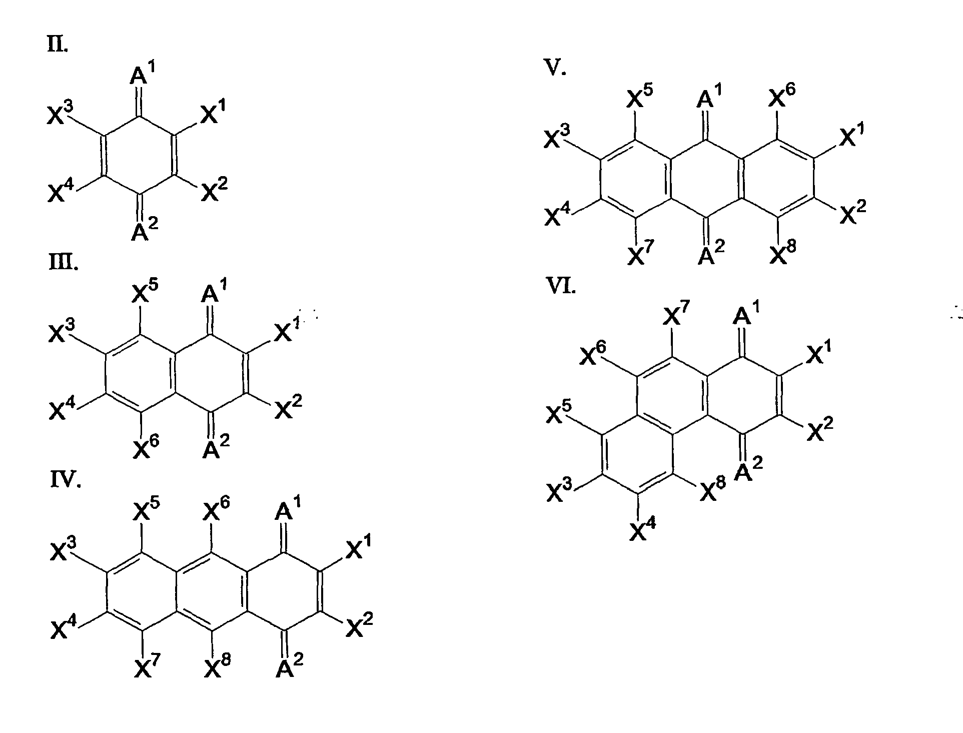

In the above formulae embodying general formula I, X is exemplified as X¹, X², X³, X⁴ to X⁸, respectively, which are each independently intermediate groups or atoms or a chemical bond, at least one of which forms the intermediate moiety that is substituted with an HTM.

In formulae I to VI, the central carbon based conjugated or aromatic moiety Y may comprise heteroatoms, e.g. N, O S, Si, Ge, replacing one or more carbon atoms.

Residues A¹ and A² are electron acceptor residues, having at least one π-electron rich bond, capable of enhancing the density of π -electrons within moiety Y. In addition to accessory residues A¹ and A², further electron donating groups may be conjugatedly linked to moiety Y. In addition to residues A¹ and A², a hole transport moiety (HTM) or more HTMs are covalently linked to moiety Y by intermediate moiety X. The at least one HTM is capable of hole or electron transport. However, the at least one HTM is not conjugatedly linked to moiety Y.

Embodiments of general formulae II to VI are preferred compounds, wherein HTM is selected from the group comprising moieties capable of hole transport, for example tris-[(*N*,*N-*diaryl)amino]-triphenylamines like 4,4',4"-tris[(*N-*(1-naphthyl)-*N-*phenyl-amino-triphenylamine] (1-TNATA) and its derivatives, 4,4',4"-tris[(*N*-(2-naphthyl)-*N-*phenyl amino)-triphenylamine] (2-TNATA) or 4,4',4"-tris[(*N-*(3-methylphenyl)-*N-*phenyl-amino)-triphenylamine] (m-TDATA) and its derivatives, 4,4',4"-tris(carbazole-9-yl)triphenylamines; *N*,*N*,*N*',*N*'-tetra arylbenzidines as *N,N,N',N'*-tetra phenyl benzidine and its derivatives, *N*,*N*'-bis(l-naphthyl)-*N*,*N*'-diphenyl-benzidine (α-NPD), *N*,*N*'-di(naphthalene-2-yl)-*N*,*N*'-diphenyl-benzidine (β-nod), 4,4'-bis(carbazole-9-yl)biphenyl (CBP) and its derivatives, and their heteroatom substituted analogs (e.g. thienyl-, selenyl-, furanyl-derivatives); 4,4'-bis(2,2'-diphenylvinyl)-1,1'-biphenyl (DPVBI); triarylamines and their derivatives, 4,4'-bis(*N*,*N* diarylamino)-terphenyls, 4,4'-bis(*N*,*N-*diarylamino)-quarterphenyls and their homologs and derivatives;
wherein A¹ and A² are independently electron donator moieties, for example selected among cyano groups, -C(CN)₂, -NCN;
wherein at least one of X¹ to X⁸ is chemically bonded to a hole transport moiety (HTM). The at least one of X¹ to X⁸ can be selected from the group comprising -O-, -S-, R-, -SiR¹R², - CR¹R² -CR¹=CR², -NR¹, -N=CR¹, -N=N-, and a chemical bond;
wherein X¹ to X⁸, which are not bonded to an HTM, can be selected independently from the group comprising -H, -F, -CN, R-, -OR¹, -SR¹, -NR¹R², -SiR¹R²R³, -CR¹R²R³, -CR¹=CR²R³, -N=NR¹, and HTM;
wherein R, R¹, R² and R³ can be selected independently from substituted or unsubstituted alkyl, vinyl, allyl and/or (hetero-)aryl and/or (hetero-) cyclic moieties, hydrogen or HTM as defined above.

When the X binding the at least one HTM to Y is a chemical bond, the compound according to the invention is a dye.

It is a specific advantage of the HTL or ETL compounds according to the present invention that they are intrinsically p-doped and, accordingly, a co-evaporation for building an HTL of a matrix material in combination with its dopant is no longer necessary. Accordingly, the production process for organic EL devices is facilitated using the HTL compounds according to the present invention. As a further effect of the intrinsically doped HTL compounds according to the present invention, HTLs are more homogenous and can be deposited with greater reproducibility in comparison to matrix compositions consisting of a matrix material and an admixed p-dopant.

A further advantage of the HTL and ETL compounds according to the invention are their higher glass transition temperatures in comparison to the system of p-doped matrix materials. The higher glass transition temperatures are assumed to result from steric effects within the HTL compounds.

A further characteristic of the ETL and ETL compounds according to the invention is their generally reduced mobility within an electric field, which is a desired property for constructing stable organic electroluminescent (EL) devices, preferably resulting in an increased long-term stability.

The inventive compounds are intrinsically doped HTLs and ETLs, respectively, which allows their deposition with greater homogeneity and reproducibility than matrix compositions consisting of a matrix material and an admixed dopant.

In a further aspect, the present invention provides a method for production of the HTL compounds, comprising the following central synthetic steps:
A) A di-substituted 1,4-cyclohexanedione moiety is reacted to a stable diketal, for example a cyclic ketal on both ketone moieties. The two substituting groups of the 1,4-cyclohexanedione moiety are chemically reactive to allow the formation of a chemical bond to an HTM. Preferred substituting groups are π-electon rich compounds, for example the substituent groups can suitably be halogenated aromatic moieties that are reactive with HTM comprising an aromatic residue.
   The linkage with at least one HTM. is obtained by reaction of the chemically reactive substituent group with a residue of the HTM, for example by reaction of a halogenated phenyl group with an aromatic residue of the HTM. The linkage can be direct between the 1,4-cyclohexanedione moiety and the HTM or via intermediate linker moieties. As a result, the cyclohexyl moiety, substituted with two diketal groups, is derivatized on its reactive substituent groups with HTMs. In a subsequent oxidation reaction, the two diketal groups are reoxidized to ketone groups.
   It is essential, that the at least one HTM is linked in a non-conjugated manner with the 1,4-cyclohexanedione moiety.
B) Following the linkage of at least one HTM to the cyclohexyl moiety substituted with two opposing ketone groups, the ketones are reacted for replacement of the ketones by electron acceptor moieties, for example cyano imine groups or a dicyano methylene group. A subsequent oxidation generates a 1,4-cyclohexanedione group, conjugated in positions 2 and 5 to two electron donating moieties. The 1,4-cyclohexanedione group is additionally substituted non-conjugatedly with at least one HTM.

In general, HTL compounds according to the present invention can be coated according to known techniques, including vapour deposition (including PVD, CVD, OVPD) and coating (spray, spin or knife coating) from a solution or sputtering, depending on the molecular weight and solubility of the compounds. In general, HTL compounds having a very high molecular weight are difficult to evaporate and, accordingly, in such cases coating from a solution is preferred. A person skilled in the art can easily determine an appropriate method for coating HTL compounds. Methods for the determination of an appropriate solvent are also commonly known. Preferred solvents are chlorobenzene, toluene and xyloles.

The present invention will now be described by way of examples, which are not intended to limit the scope of the invention. Reference is made to the figures, wherein
- Figure 1 schematically depicts an organic field electric transistor (OFET) in cross-section. Therein, the layer designated as semiconductor illustrates the position of an HTL according to the invention,
- Figure 2 schematically depicts an OLED in cross-section with the HTL being formed of the compounds according to the present invention,
- Figure 3 schematically depicts an inverted OLED in cross-section with the HTL in form of the compounds according to the invention,
- Figure 4 schematically depicts a solar cell in cross-section with the p-type layer semiconductor being formed of a compound according to the present invention,
- Figure 5 schematically shows the steps for synthesis of exemplary compounds according to the invention,
- Figure 6 schematically shows the steps for synthesis of inventive compound **19,**
- Figure 7 schematically shows the structure comprising an inventive compound as a charge transport layer,
- Figure 8 shows the electrical behaviour of 1-TNATA in comparison to 1-TNATA doped with inventive compound **19,** and
- Figure 9 shows the electrical behaviour of inventive compound **19.**

### Example 1: OLED comprising inventive HTL, vacuum deposited

This example describes the structure of an inverted OLED, schematically depicted in Figure 3, however, a non-inverted structure, e.g. schematically depicted in Figure 2, can be realized using the compounds according to the invention as well. For a non-inverted OLED structure, compound **14** and/or compound **19,** obtainable according to Example 3, was vacuum deposited onto an ITO covered glass substrate up to a layer thickness of 10 - 500 nm. Subsequently, an electron blocking layer was vacuum deposited, followed by vacuum deposition of an emissive layer (Alq₃), a hole blocking layer (BCP) and an electron transport layer (TAZ). The cathode (LiF/Al) was deposited as the final layer.

For an inverted OLED structure, after deposition of a cathode on a substrate, an electron transport layer, followed by an optional hole blocking layer, an electroluminescent layer and an optional electron blocking layer, the inventive compound was deposited to form the hole transport layer as a dopant in 1-TNATA or as a pure substance. Deposition of the hole transport layer was followed by vacuum deposition of a protective layer (pentacene) before deposition of poly(3,4-[ethylenedioxy]-thiophene) (PEDT) with poly(styrene sulfonic acid) (PSS), also known as PEDT:PSS (e.g. Baytron P^{®}, before applying ITO as the cathode.

In the alternative to a hole transport layer comprising the compound according to the invention as a dopant, the inventive compound can form the hole transport layer as the only component of this layer.

Accordingly, in a further embodiment, compound **14** and/or **19** can form an injection layer, superseding the need for PEDT:PSS as an injection layer. As a consequence, the pentacene protective layer can be omitted. This represents a specific advantage of the compounds according to the invention, especially because the injection layer can be applied in a vacuum process, e.g. without interrupting the vacuum processing to coat the final electrode layer. Further, the specific advantage of the compounds according to the invention to obviate the need for an injection layer (e.g. PEDT:PSS) and a protective layer (e.g. pentacene) allows for a more simple structure of the structure of the organoelectric device. Prior to the invention, the protective layer was required to allow the coating of the stacked sensitive organoelectric compounds using wet-chemical processing to allow the coating of highly conductive compounds like PEDT:PSS.

### Example 2: OLED comprising inventive HTL, deposited by spin coating

Example 1 was repeated except that compound **14,** or alternatively compound **19,** obtained according to example 3, was deposited by spin coating from a solution in (solvent) to a final layer thickness of 10-500 nm.

The electric and EL properties essentially corresponded to those found in Example 1.

### Example 3: Electric properties of 2.5-bis-(4-diphenylaminobenzyl)-1,4-bis-(dicyanomethylidene)-cyclohexa-2,5-diene (19)

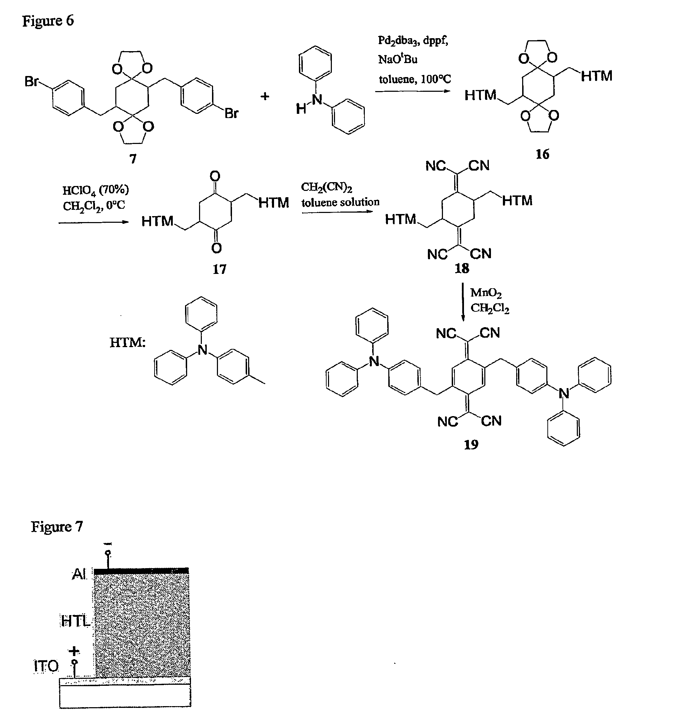

The synthesis of compound **19** is schematically shown in Figure 6. Synthesis of compound **19** was essentially analogous to synthesis of **14,** described in example 4. The melting point of compound **19** is 252 °C.

As an example for the compounds according to the invention, compound **19,** shown in Figure 6, was used as a p-dopant within a 100 nm thickness layer of 1-TNATA at concentrations of 1.2 vol-% and 2.5 vol-%, respectively in comparison to undoped 1-TNATA. 1-TNATA doped with compound **19** was coated onto ITO-covered glass substrate and covered by an electrically conductive aluminum layer.

The structure of the measuring set-up, and of a simple electroorganic device, respectively, are depicted in Figure 7. As can be taken from Figure 7, the inventive compounds can form a layer in direct contact with both electrode surfaces without any need for an additional injection layer when used as a dopant in admixture with 1-TNATA or as a pure substance, i.e. without additional matrix material. This structure is sufficient for transporting charge from one electrode to the other.

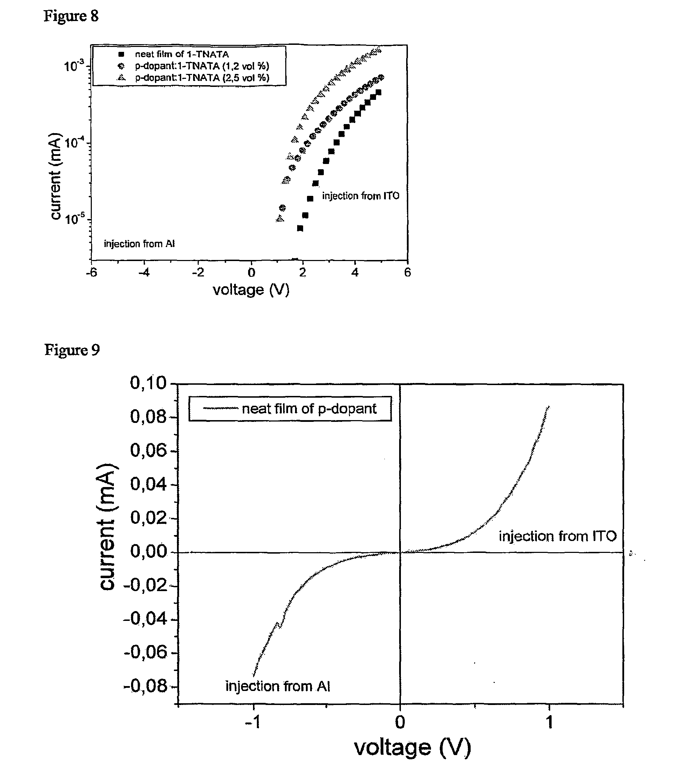

The electric properties of a charge transport layer comprising an inventive substance as a dopant of matrix material (1 -TNATA) are shown in Figure 8, demonstrating that increasing concentrations of compound **19** result in a dramatically increased positive charge transport (I[A]) in response to increasing voltage (U[V]), i.e. when charge was injected from the ITO layer. When voltage was reversed to inject charge from the A1 layer, no conductivity was measured.

As an alternative to using the inventive compound as a p-dopant, the electrically conductive layer was formed of compound **19** (neat film of p-dopant) by itself within a structure according to Figure 7, i.e. without further matrix compounds in admixture. For measurements, the polarity of electrodes was reversed if necessary. Compound **19** was layered onto ITO glass substrate and covered with aluminum as above. When applying voltage to inject charge from the ITO layer, the response is a rapid increase in positive charge transport, whereas reversing the voltage to inject charge from the A1 layer results in a rapid increase of negative charge transport. This behaviour, shown in Figure 9, is proof for the suitability of the inventive compounds for forming electrically conductive layers in FETs.

This example demonstrates that compounds according to the invention can form a p-dopant in hole transport layers and, alternatively, that they can form hole transport layers and electron transport layers without further matrix materials added. Further, this is proof for the applicability of the inventive substances as hole and/or electron injection layers. As a specific advantage, a conductive layer formed of the compounds according to the invention yields a very homogenous and evenly distributed phase.

### Example 4: Synthesis of 2.5-bis{4-[(4'-diphenylamino-biphenyl-4-yl)-phenylamino]-benzyl}-1,4-bis(dicyanomethyliden)-cyclohexa-2,5-diene (14)

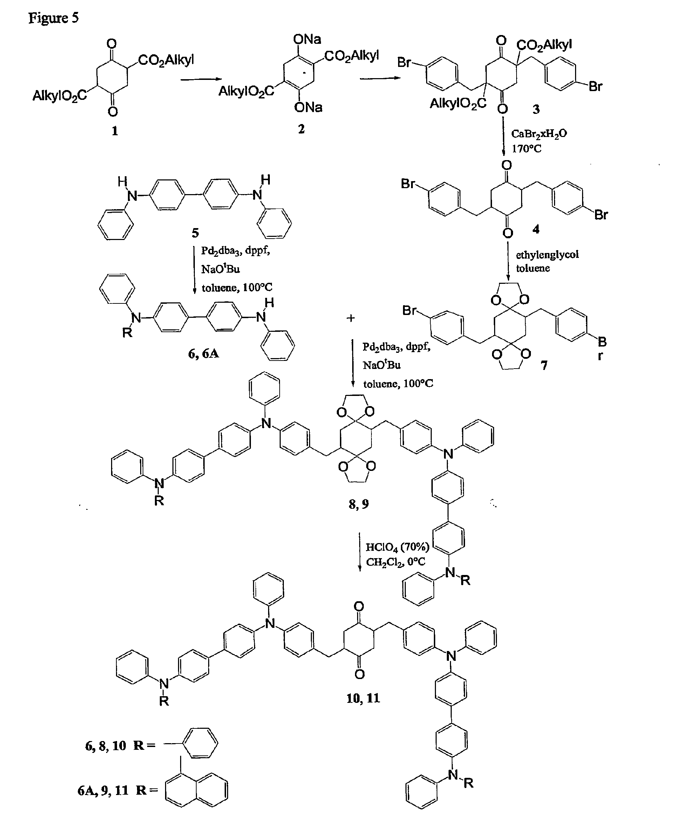

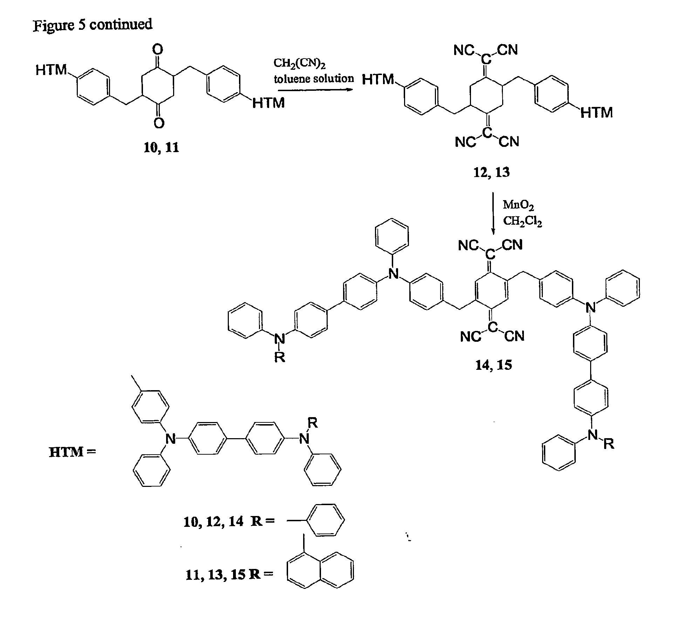

The synthesis of 2,5-bis{4-[(4'-diphenylamino-biphenyl-4-yl)-phenylamino]-benzyl}-1,4-bis(dicyanomethyliden)-cyclohexa-2,5-diene **(14)** is schematically depicted in Figure 5.
In this example, the 4'-bromobenzyl substituents represent the two substituting groups of the 1,4-cyclohexanedione moiety that are chemically reactive to allow the later formation of a chemical bond to an HTM.

Starting from 2,5-bis(methoxycarbonyl)-cyclohexa-1,4-dione **(1),** 2,5-bis(methoxycarbonyl)-2,5-bis(4-bromobenzyl)-cyclohexa-1,4-dione **(3)** is accessible via intermediate compound **2** by reacting it with 4-bromobenzylbromide. Analytical results for compound **3** are MS (EI, 70 eV): m/z (%) = 566 (10) [M⁺], EA: calc. C= 50.91, H= 3.91, Br= 28.22; measured C= 51.25, H = 3.95, Br = 28.07.

Removal of the two ester groups in 2- and 5- positions is obtained by heating compound **3** in the presence of calcium bromide. In detail, 2,5-bis(4-bromobenzyl) cyclohexa-1,4-dione **(4)** is obtained by stirring 1.13 g (2 mmol) 2,5-bis(methoxycarbonyl)-2,5-bis(4-bromobenzyl)-cyclohexa-1,4-dione **(3)** in mixture with 2.80 g calcium bromide at 180 °C under an inert gas atmosphere in a 250 mL three-necked flask equipped with a reflux condenser for 3 hours. Then, 100 mL of 1 N HCl were added. Phases were separated and the aqueous phase was extracted four times with 15 mL methylene chloride. The organic phase is dried over sodium sulfate and the solvent is removed in a rotary evaporator. The solid product is purified by Soxhlet extraction using diethyl ether. 0.72 g (1.6 mmol) of a white solid are obtained as the isomeric mixture. Analytical results for 4 are MS (EI, 70 eV): m/z (%) = 450 (40) [M⁺] EA: calc. C = 53.36, H =4.03, Br = 35.50; measured C = 53.74, H=4.07, Br = 35.45, melting point m.p. =158-160 °C.

The conversion of 4 to 2,5-bis(4-bromobenzyl)-1,4,9,12-tetraoxa-dispiro[4.2.4.2]tetradecane (7) was performed according to *Liebigs Ann. Chem.* 186-190 (1982). MS (EI, 70 eV): m/z (%) = 538 (16) [M⁺]. EA: calc. C = 53.55, H = 4.87, Br = 29.69; measured C = 55.33, H = 4.94, Br = 29.10, melting point m.p. = 200-202 °C.

As a representative HTM, *N*,*N'N'*-triphenyl-biphenyl-4,4'-diamine **(6)** is obtainable from compound 5 via the Buchwald coupling, using a reaction in toluene at 100°C according to J.Am.Chem.Soc. 118,7215-7216 (1996) and J.Am.Chem.Soc. 62, 1568-1569 (1997). Compound **6** is purified by flash column chromatography (n-hexane : ethyl acetate (10: 1), Rf= 0.3).

2,5-bis{4-[4'-diphenylamino-biphenyl-4-yl)-phenyl-amino]-benzyl}-1,4,9,12-tetraoxadispiro[4.2.4.2]tetradecane **(8)** is isolated as a white solid from the reaction of **6** with **7.** The melting point of **8** was determined to 142-143 °C. ESI-MS (CH₃CN/toluene): 1201 [M⁺]. Elementary analysis (EA): calc. C = 83.97, H = 6.04, N = 4.66; measured C = 83.87, H = 6.12, N=4.42.

In the alternative, 2,5-bis(4-{[4'-(naphthalene-1-yl-phenyl-amino)-biphenyl-4-yl]-amino}-benzyl)-1,4,9,12-tetraoxa-dispiro[4.2.4.2]tetradecane **(9)** is obtained as a white solid from the reaction of **6A** with **7.** The melting point was determined to 130-132 °C. ESI-MS (CH₃CN/ toluene): m/z =1301 [M⁺]
EA: calc. C = 84.88, H = 5.90, N = 4.30; measured C = 84.72, H = 6.24, N = 3.78.

2,5-bis(4-{[4'-diphenylamino-biphenyl-4-yl]-phenylamino]-benzyl}-cyclohexane-1,4-dione **(10)** was obtained by cooling 0.6 g (0.5 mmol) of compound **8,** dissolved in 70 mL dichloromethane in a 250 mL two-necked flask under inert gas atmosphere to 0 °C. Slowly, 0.5 mL 70% HClO₄ are added dropwise and stirring continued at 0 °C for one hour. After 3 hours, the reaction solution is neutralised with 100 mL saturated NaHCO₃ and stirred for one hour at room temperature. The organic phase is dried over magnesium sulfate and the solvent is removed on a rotary evaporator. The residue is purified by flash column chromatography in methylene chloride : n-hexane (5 : 1). 0.185 mg (1.6 mmol) of a white solid are obtained as an isomeric mixture having a melting point of 142 °C and 136 °C, respectively.

Isomer 2: ¹H NMR (200 MHz, CDCl₃): δ = 7.36-6.87 (m, 54H, H_{ar.}), 3.18 (dd, *J* = 13.9 and 4.2 Hz, 2H, H_{cyc.}), 3.09-2.71 (m, 2H, H_{cyc.}), 2.62 (dd, J =17.7 and 5.9 Hz, 2H, H_{cyc.}), 2.49-2.21 (m, 4H, H_{methylene}).
¹³C NMR (50 MHz, CDCl₃): δ = 209.3 (C_{C=O}), 147.7-146.4 (Cₐᵣ.), 134.8-122.8 (C_{ar.}), 48.3 (C_{cyc.,CH}), 41.4 (C_{cyc., CH2}), 34.5 (C_{methylene}).
ESI-MS (CH₃CN/toluene): m/z =1112 [M⁺]

In the alternative to compound **10,** 2,5-bis(4-{[4'-(naphtalene-1-yl-phenylamino)-biphenyl-4-yl]-phenylamino}-benzyl)-cyclohexa-1,4-dione **(11)** is obtained by the same synthetic steps as compound **10** above when starting from compound **9** instead of compound **8.** Compound **11** is obtained as a white solid (isomeric mixture).

Compounds **10** and **11,** respectively, are reacted to 2,5-bis-{4-[(4'-diphenylamino-biphenyl-4-yl)-phenylamino]-benzyl}-1,4-bis-(dicyanomethylidene)-cyclohexane (**12**) and 2,5-bis-(4-{[4'-(naphthalene-1-yl-phenylamino)-biphenyl-4-yl)-phenylamino}-benzyl)-1,4-bis-(dicyanomethylidene)-cyclohexane **(13)** by stirring 0.9 mmol of compound **10** and **11,** respectively, (0.99 g compound 10) in mixture with 0.178 g (2.7 mmol) CH₂(CN)₂ and a catalytic amount of beta-alanine 10 mL alcoholic toluene solution, with the alcohol preferably being methanol, ethanol or propanol, in a 50 mL one-necked flask, equipped with a reflux condenser. After 72 hours of stirring at 80 °C, compounds **12** and **13,** respectively, are removed by filtration and washed with ethanol. There are obtained 0.85 g (0.7 mmol) of compound **12** as a pale yellow solid having a melting point of 278-280 °C.

Compound **12:** ¹H NMR (200 MHz, CDCl₃): δ = 7.48-7.01 (m, 54H, Hₐᵣ.), 3.71 (dd, 2H, H_{cyc.}), 3.22 (d, 2H, H_{cyc.}), 2.79-2.61 (m, 6H, H_{methylene and cyc.}).
¹³C NMR (50 MHz, CDCl₃): δ = 176.9 (C_{C=C(CN)}), 147.9-146.5 (Cₐᵣ.), 135.5-123.0 (C_{ar.}), 110.9 (C_{CN}), 110.7 (C_{CN}), 88.5 (C_{C=C(CN)}), 45.6 (C_{cyc.,CH}), 39.9 (C_{cyc.,CH2}), 35.4 (C_{methylene}).
ESI-MS (CH₃CN/toluene): m/z =1208 [M⁺]
EA: calc. C = 85.40, H = 5.33, N = 9.26; measured C = 84.92, H = 5.32, N = 8.90

Compound **13** is isolated as a yellow solid having a melting point of 156-158 °C.

Compound **13:** ¹H NMR (200 MHz, CDCl₃): δ = 7.97-7.76 (m, 7H, H_{naphthyl}),7.52-6.90 (m, 53H, Hₐᵣ.), 3.68 (dd, 2H, H_{cyc.}), 3.21 (d, 2H, H_{cyc.}), 2.84-2.70 (m, 6H, H_{methylene and cyc.}).
¹³C NMR (50 MHz, CDCl₃): δ = 176.9 (C_{C=C(CN)}), 148.5-1143.6 (Cₐᵣ.), 135.6-122.0 (Cₐᵣ.), 111.0 (C_{CN}), 110.7 (C_{CN}), 88.4 (C_{C=C(CN)}), 45.6 (C_{cyc.,CH}), 40.0 (C_{cyc.,CH2}), 34.1 (C_{methylene}). ESI-MS (CH₃CN/toluene): m/z =1309 [M⁺].
EA: calc. C = 86.21, H = 5.23, N = 8.56; measured C = 86.10, H = 5.45, N = 7.80

### 2,5-bis{4-[(4'-diphenylamino-biphenyl-4-yl)-phenylamino]-benzyl)-1,4-bis-(dicyanomethylidene)-cyclohexa-2,5-diene (14)

In a 250 mL two-necked flask there are dissolved 0.2 g (0.2 mmol) of compound **12** in 50 mL of dichloromethane. To the resultant solution, 0.55 g activated manganese dioxide is added. The mixture is stirred at room temperature under inert gas atmosphere for 3 h and is then filtered over silica gel. The solvent is removed and the residue is recrystallized from a mixture of toluene and acetonitrile at -10 °C. There are obtained 0.12 g (0.1 mmol) of compound **14** as a green solid with a melting point of 177-179°C.

¹H NMR (400 MHz, CDCl₃): δ = 6.99-7.46 (m, 56H, H_{cyc. and ar.}), 4.27 (s, br., 4H, H_{methylene}), ¹³C NMR (100 MHz, CDCl₃): δ = 150.3 (C_{C=C(CN)}), 147.7-122.8 (Cₐᵣ), 143.6 (C_{cyc., C=CH}), 124(C_{cyc.,C=CH}), 112.7 (C_{CN}) 113.9 (C_{CN}), 87.1 (C_{C=-C(CN)}), 38.4 (C_{methylene}). ESI-MS (CH₃CN/toluene): m/z = 1204 [M⁺]. Elementary analysis: calc.: C = 85.69, H = 5.02, N = 9.30; measured: C = 85.54, H = 5.56, N = 8.54.

## Claims

1. Use of a compound as a dopant in a hole or electron transport layer and/or in a hole or electron injection layer for electrooptic devices, **characterized by** the compound of formula I 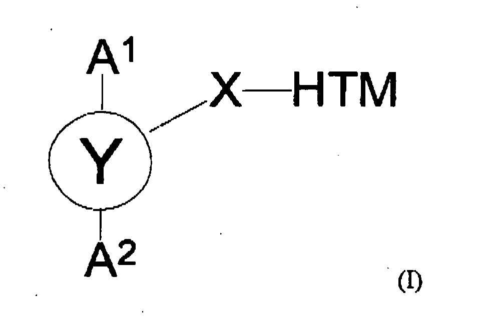 wherein moiety Y represents a central carbon based structure with accessory residues A¹ and A² each conjugatedly linked to Y, wherein residues A¹ and A² are electron accepting residues and wherein HTM is a hole transport moiety which is non-conjugatedly linked to moiety Y and wherein X are intermediate groups or a chemical bond.

2. Use according to claim 1, wherein X is a chemical bond.

3. Use according to one of the preceding claims, wherein Y comprises a five- or six-membered ring which conjugatedly links residues A¹ and A².

4. Use according to claim 3, wherein the five- or six-membered ring comprises at least one heteroatom.

5. Use according to one of the preceding claims, **characterized in that** formula I is one of the following formulae: 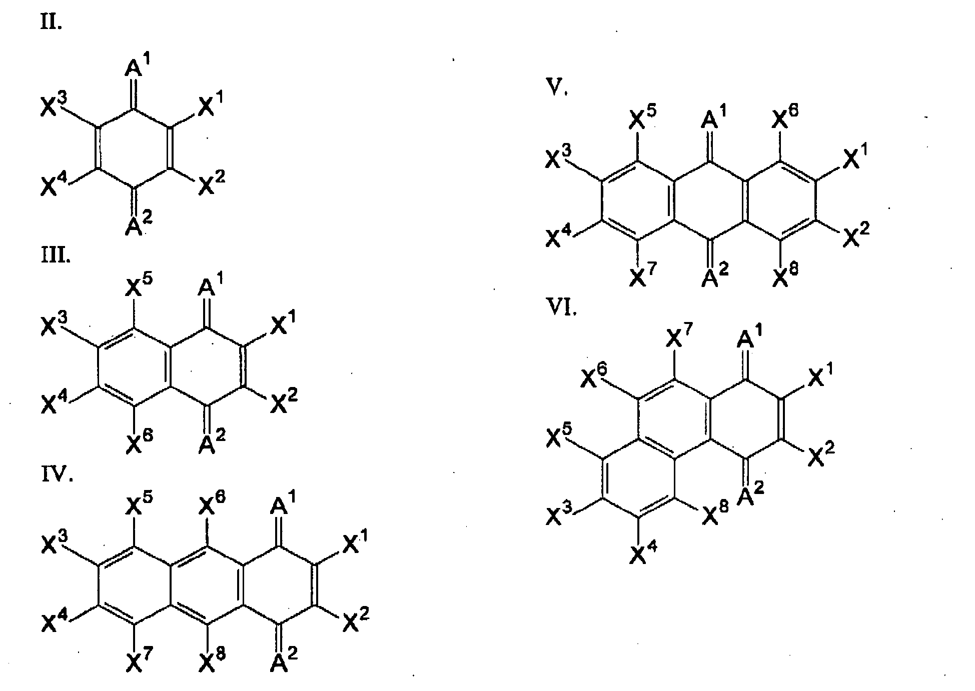 or

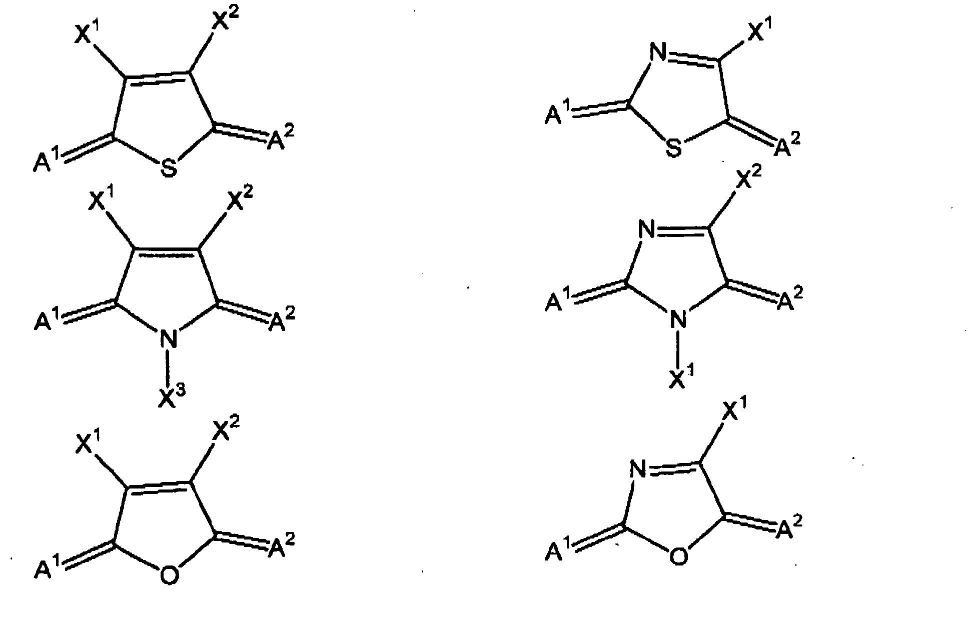

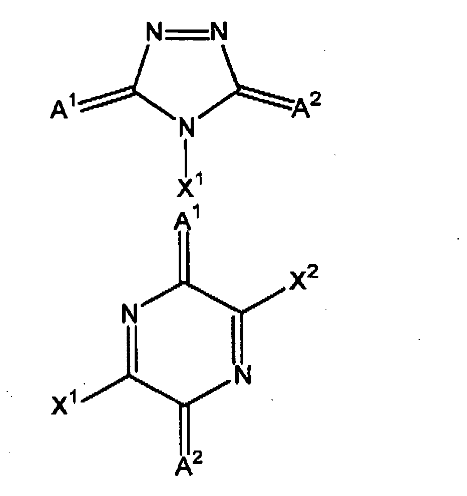

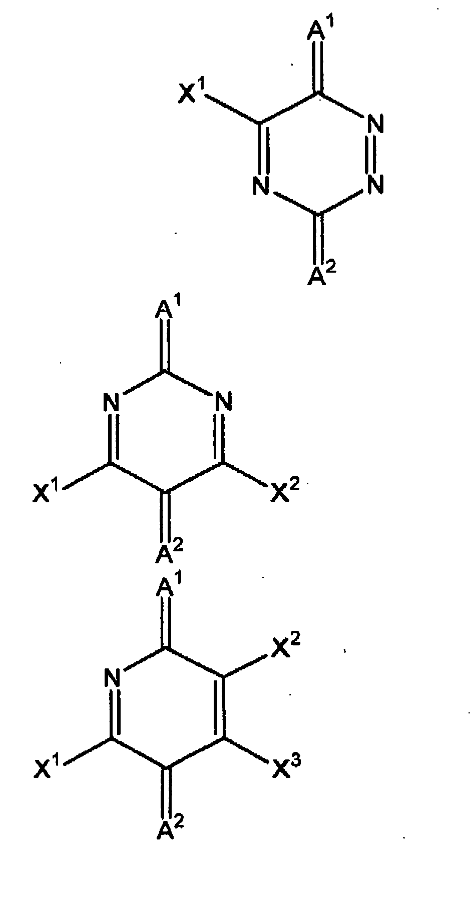

6. Use according to one of the preceding claims, **characterized in that** 1 to 8 HTM are comprised, wherein each HTM is linked to one of X¹, X², X³, X⁴ to X⁸.

7. Use according to one of the preceding claims, **characterized in that** X¹, X², X³, X⁴ to X⁸ which are substituted with HTM are selected from the group comprising -O-, -S-, - SiR¹R², -CR¹R², -CR¹=CR², -NR¹, -N=CR¹, -N=N-, and a chemical bond.

8. Use according to one of the preceding claims, **characterized in that** X¹, X², X³, X⁴ to X⁸ which are not substituted with HTM are selected from the group comprising -H, -F, -CN, -OR¹, -SR¹, -NR¹R², -SiR¹R²R³, -CR¹R²R³, -CR¹=CR²R³, -N=NR¹, and HTM.

9. Use according to one of the preceding claims, **characterized in that** HTM is selected from the group comprising tris-[(*N*,*N-*diaryl)amino]-triphenylarnines, 4,4',4"-tris[(*N-*(1-naphthyl)-*N-*phenyl-amino-triphenylamine] (1-TNATA) and its derivatives, 4,4',4"-tris[(*N*-(2-naphthyl)-*N*-phenyl amino)-triphenylamine] (2-TNATA); 4,4',4"-tris[(*N*-(3-methylphenyl)-*N*-phenyl-amino)-triphenylamine] (m-TDATA) and its derivatives, 4,4',4"-tris(carbazole-9-yl)triphenylamines; *N,N,N',N'*-tetra arylbenzidines as *N*,*N*,*N*',*N*'-tetra phenyl benzidine and its derivatives, *N*,*N*'-bis(1-naphthyl)-*N*,*N*'-diphenyl-benzidine (α-NPD), *N*,*N'*-di(naphthalene-2-yl)-*N*,*N*"-diphenyl-benzidine (β-NPD), 4,4'-bis(carbazole-9-yl)biphenyl (CBP) and its derivatives; 4,4'-bis(2,2'-diphenylvinyl)-1,1'-biphenyl (DPVBI); triarylamines and their derivatives, 4,4'-bis(*N*,*N*-diarylamino)-terphenyls, 4,4'-bis(*N*,*N*-diarylamino)-quarterphenyls and their homologs and derivatives.

10. Use according to claim 6, **characterized in that** the HTM is a heteroatom substituted analog thereof.

11. Use according to claim 6, **characterized in that** the heteroatom substituted analog is a thienyl-, selenyl- or furanyl-derivative.

12. Use according to one of the preceding claims, **characterized in that** A¹ and A² are independently selected from the group comprising cyano groups, -C(CN)₂, and -NCN.

13. Use according to one of the preceding claims, **characterized in that** the compound is 2,5-bis{4-[(4'-diphenylamino-biphenyl-4-yl)-phenylamino]-benzyl)-1,4-bis-(dicyanomethylidene)-cyclohexa-2,5-diene **(14).**

14. Use according to one of the preceding claims, **characterized in that** the compound is 2,5-bis-(4-diphenylaminobenzyl)-1,4-bis-(dicyanomethylidene)-cyclohexa-2,5-diene **(19).**

15. Use according to one of the preceding claims as a hole or electron transport layer and/or as a hole or electron injection layer without further matrix materials added.

16. Process for synthesizing a compound for use according to one of the preceding claims, **characterized by** the steps of
a) reacting a di-substituted 1,4-cyclohexanedione moiety to a stable diketal,
b) forming a non-conjugated chemical bond between the 1,4-cyclohexanedione moiety to at least one HTM,
c) chemically replacing the ketone groups for electron donating moieties to form a conjugated bond between the electron donating moieties and the cyclohexyl moiety, and
d) oxidizing 1,4-bis(dicyanomethylidene)-cyclohexane to 1,4-bis(dicyanomethylidene)-1,4-cyclohexa-2,5-diene.

17. Electric device, which is an OLED, OFET, laser or photovoltaic device, **characterized by** the use of a compound according to one of claims 1 to 16,

18. Process for producing electric or electro-optic devices, **characterized by** comprising a compound according to one of claims 1 to 15.

19. Process according to claim 18, **characterized in that** the organic layers of the device and the final contacting electrode are formed in a vacuum process.

20. Process according to claim 19, **characterized in that** the vacuum process is a PVD (physical vapour deposition), CVD (chemical vapour deposition) or an OVPD (organic vapour physical deposition) process.

21. Process according to claim 18, **characterized in that** the compound used according to one of claims 1 to 15 is applied by coating from solution or sputtering.

22. Process according to claim 21, **characterized in that** the coating is spray, spin, dip or knife coating.

## Patentansprüche

1. Verwendung einer Verbindung als ein Dotierungsmittel in einer Loch- oder Elektronentransportschicht und/oder in einer Loch- oder Elektroneninjektionsschicht für elektrooptische Vorrichtungen, **gekennzeichnet durch** die Verbindung nach Formel I 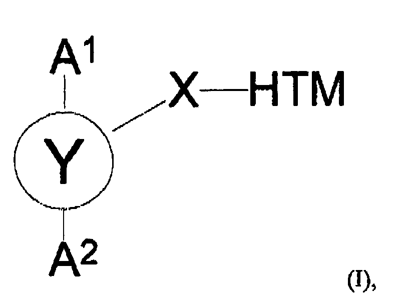 bei der Rest Y ein zentrale Struktur auf Kohlenstoffbasis mit akzessorischen Resten A¹ und A² darstellt, die jeweils konjugiert an Y gebunden sind, wobei die Reste A¹ und A² elektronenakzeptierende Reste sind und bei der HTM eine Lochtransportgruppe ist, welche nicht-konjugiert an Gruppe Y gebunden ist und bei der X zwischengestellte Gruppen oder eine chemische Bindung ist.

2. Verwendung nach Anspruch 1, bei der X eine chemische Bindung ist.

3. Verwendung nach einem der voranstehenden Ansprüche, bei der Y einen fünf- oder sechsgliedrigen Ring umfasst, der die Reste A¹ und A² konjugiert verbindet.

4. Verwendung nach Anspruch 3, bei der der fünf- oder sechsgliedrige Ring wenigstens ein Heteroatom umfasst.

5. Verwendung nach einem der voranstehenden Ansprüche, **dadurch gekennzeichnet, dass** Formel I eine der folgenden Formeln ist: 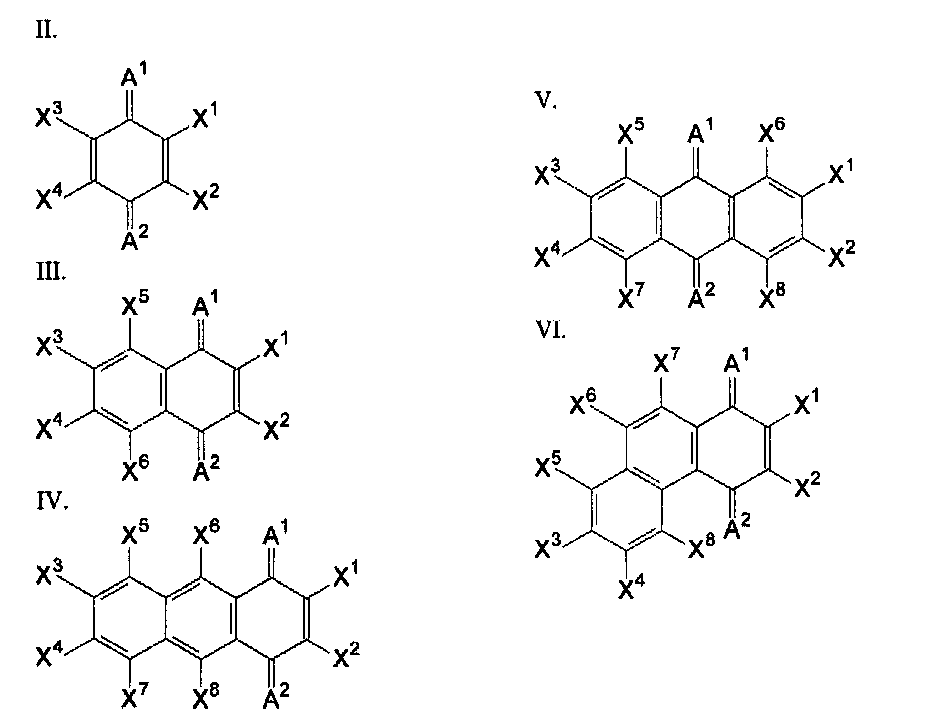 or

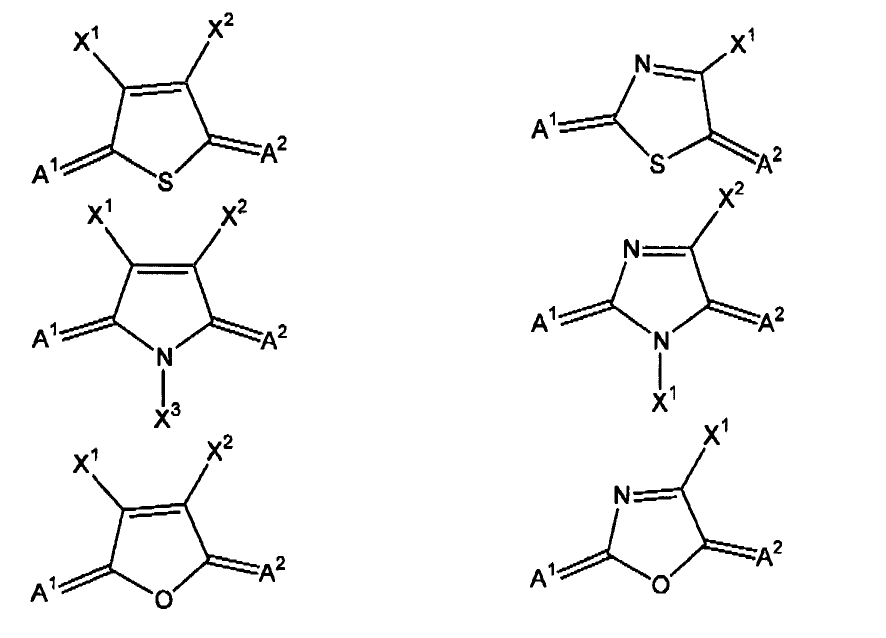

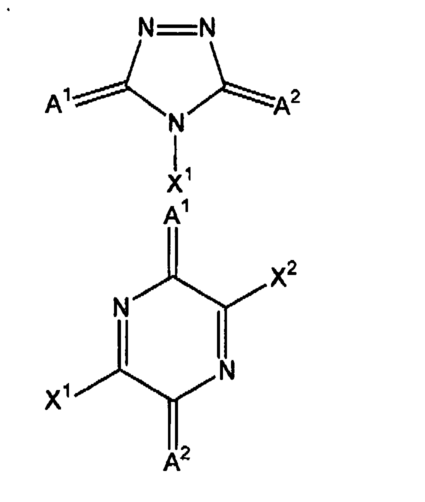

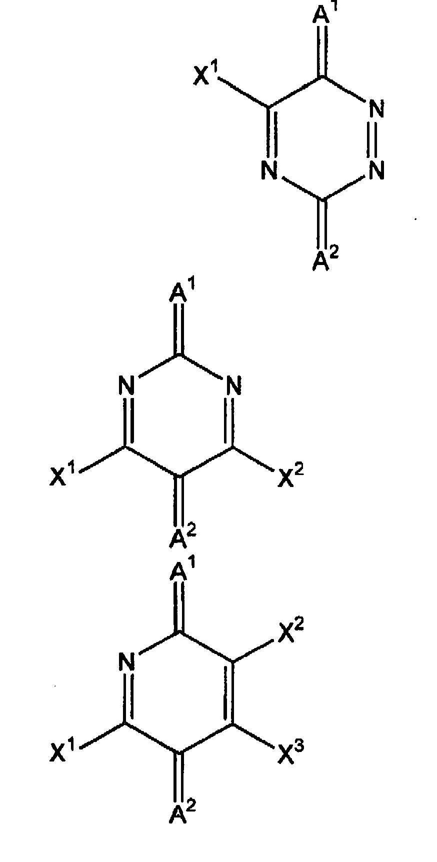

6. Verwendung nach einem der voranstehenden Ansprüche, **dadurch gekennzeichnet, dass** 1 bis 8 HTM umfasst sind, wobei jede HTM an einen von X¹, X², X³, X⁴ bis X⁸ gebunden ist.

7. Verwendung nach einem der voranstehenden Ansprüche, **dadurch gekennzeichnet, dass** X¹, X², X³, X⁴ to X⁸, welche mit HTM substituiert sind, aus der Gruppe ausgewählt sind, die -O-, -S-, -SiR¹R², -CR¹R², -CR¹=CR². -NR¹, -N=CR¹, -N=N- und eine chemische Bindung umfasst.

8. Verwendung nach einem der voranstehenden Ansprüche, **dadurch gekennzeichnet, dass** X¹, X², X³, X⁴ to X⁸, welche nicht mit HTM substituiert sind, aus der Gruppe ausgewählt sind, die -H, -F, -CN, -OR¹, -SR¹, -NR¹R², -SiR¹R²R³, -CR¹R²R³, - CR¹=CR²R³, -N=NR¹ und HTM umfasst.

9. Verwendung nach einem der voranstehenden Ansprüche, **dadurch gekennzeichnet, dass** HTM aus der Gruppe ausgewählt ist, die Tris-[(*N*,*N*-diaryl)amino]-triphenylamine, 4,4',4"-Tris[(*N*-(1-naphthyl)-*N*-phenyl-amino-triphenylamin] (1-TNATA) und dessen Derivate, 4,4',4"-Tris[(*N*-(2-naphthyl)-*N*-phenyl amino)-triphenylamin] (2-TNATA); 4,4',4"-Tris[(*N*-(3-mcthylphenyl)-*N*-phenyl-alnino)-triphenylamin] (m-TDATA) und dessen Derivate, 4,4',4"-Tris(carbazol-9-yl)triphenylamine; *N*,*N*,*N*',*N*'-Tetraarylbenzidine wie *N*,*N*,*N*',*N*'-Tetraphenyl benzidin und dessen Derivate, *N*,*N*'-Bis(1-naphthyl)-*N*,*N*'-diphenyl-benidin (α-NPD), *N*,*N'-*Di(naphthalen-2-yl)-*N*,*N*'-diphenyl-benzidin (β-NPD), 4,4'-Bis(earbazol-9-yl)biphenyl (CBP) und dessen Derivate; 4,4'-Bis(2,2'-diphenylvinyl)-1,1'-biphenyl (DPVBI); Triarylamine und deren Derivate, 4,4'-Bis(*N*,*N*-diarylamino)-terphenyle, 4,4'-Bis(*N*,*N*-diarylamino)-quarterphenyle und deren Homologe und Derivate umfassst.

10. Verwendung nach Anspruch 6, **dadurch gekennzeichnet, dass** HTM ein Heteroatomsubstituiertes Analogon dessen ist.

11. Verwendung nach Anspruch 6, **dadurch gekennzeichnet, dass** das Heteroatomsubstituierte Analogon ein Thienyl-, Selenyl- oder Furanylderivat ist.

12. Verwendung nach einem der voranstehenden Ansprüche, **dadurch gekennzeichnet, dass** A¹ and A² jeweils unabhängig aus der Gruppe ausgewählt sind, die Cyanogruppen, -C(CN)₂ und -NCN umfasst.

13. Verwendung nach einem der voranstehenden Ansprüche, **dadurch gekennzeichnet, dass** die Verbindung 2,5-Bis{4-[(4'-diphenylamino-biphenyl-4-yl)-phenylamino]-bcnzyl)-1,4-bis-(dicyanomethyliden)-cylohexa-2,5-dien **(14)** ist.

14. Verwendung nach einem der voranstehenden Ansprüche, **dadurch gekennzeichnet, dass** die Verbindung 2,5-Bis-(4-diphenylaminobenzyl)-1,4-bis-(dicyanomethyliden)-cyclohexa-2,5-dien **(19)** ist.

15. Verwendung nach einem der voranstehenden Ansprüche als eine Loch- oder Elektronentransportschicht und/oder als eine Loch- oder Elektroneninjektionsschicht ohne weitere zugesetzte Matrixverbindungen.

16. Verfahren zur Synthese einer Verbindung zur Verwendung nach einem der voranstehenden Ansprüche, **gekennzeichnet durch** die Schritte von
a) Umsetzen einer di-substituierten 1,4-Cyclohexandiongruppe zu einem stabilen Diketal,
b) Bilden einer nicht-konjugierten chemischen Bindung zwischen der 1,4-Cyclohexandiongruppe zu wenigstens einer HTM,
c) chemisches Ersetzen der Ketongruppen **durch** elektronenabgebende Gruppen, um eine konjugierte Bindung zwischen den elektronenabgebenden Gruppen und der Cyclohexylgruppe zu bilden und
d) Oxidieren des 1,4-Bis(dicyanomethyliden)-cyclohexans zu 1,4-Bis(dicyano-methyliden)-1,4-cyclohexa-2,5-dien.

17. Elektrische Vorrichtung, die eine OLED, OFET, Laser oder photovoltaische Vorrichtung ist, **gekennzeichnet durch** die Verwendung einer Verbindung nach einem der Ansprüche 1 bis 16.

18. Verfahren zur Herstellung elektrischer oder elektro-optischer Vorrichtungen, **dadurch gekennzeichnet, dass** es eine Verbindung nach einem der Ansprüche 1 bis 15 umfasst.

19. Verfahren nach Anspruch 18, **dadurch gekennzeichnet, dass** die organischen Schichten der Vorrichtung und die letzte kontaktierende Elektrode in einem Vakuumverfahren gebildet werden.

20. Verfahren nach Anspruch 19, **dadurch gekennzeichnet, dass** das Vakuumverfahren ein PVD- (physikalisches Dampfabscheiden), CVD- (chemisches Dampfabscheiden) oder ein OVPD- (physikalisches Abscheiden organischen Dampfes) Verfahren ist.

21. Verfahren nach Anspruch 18, **dadurch gekennzeichnet, dass** die gemäß einem der Ansprüche 1 bis 15 verwendete Verbindung durch Beschichten aus Lösung oder Sputtern aufgetragen wird.

22. Verfahren nach Anspruch 21, **dadurch gekennzeichnet, dass** das Beschichten Sprüh-, Rotations-,Tauch- oder Rakelbeschichten ist.

## Revendications

1. Utilisation d'un composé en tant que dopant dans couche de transport de trous ou d'électrons et/ou dans une couche d'injection de trous ou d'électrons pour dispositifs électro-optiques, **caractérisée par** le composé de formule 1 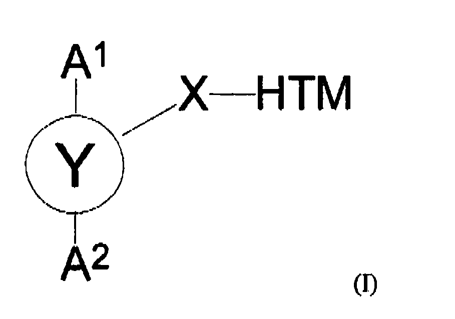 dans laquelle le résidu Y représente une structure centrale basée sur le carbone avec des résidus accessoires A¹ et A², chacun lié de façon couplée à Y, sachant que les résidus A¹ et A² sont des résidus acceptant des électrons et que HTM est un groupe de transport de trous qui est relié de façon non-couplée au groupe Y, et dans lequel X est un groupe intermédiaire ou une liaison chimique.

2. Utilisation selon la revendication 1, dans laquelle X est une liaison chimique.

3. Utilisation selon une des revendications précédentes, dans laquelle Y comprend un anneau à cinq ou six membres qui relie de façon couplée les résidus A¹ et A².

4. Utilisation selon la revendication 3, dans laquelle l'anneau à cinq ou six membres comprend au moins un hétéroatome.

5. Utilisation selon une des revendications précédentes, **caractérisée par le fait que** la formule I est une des formules suivantes : 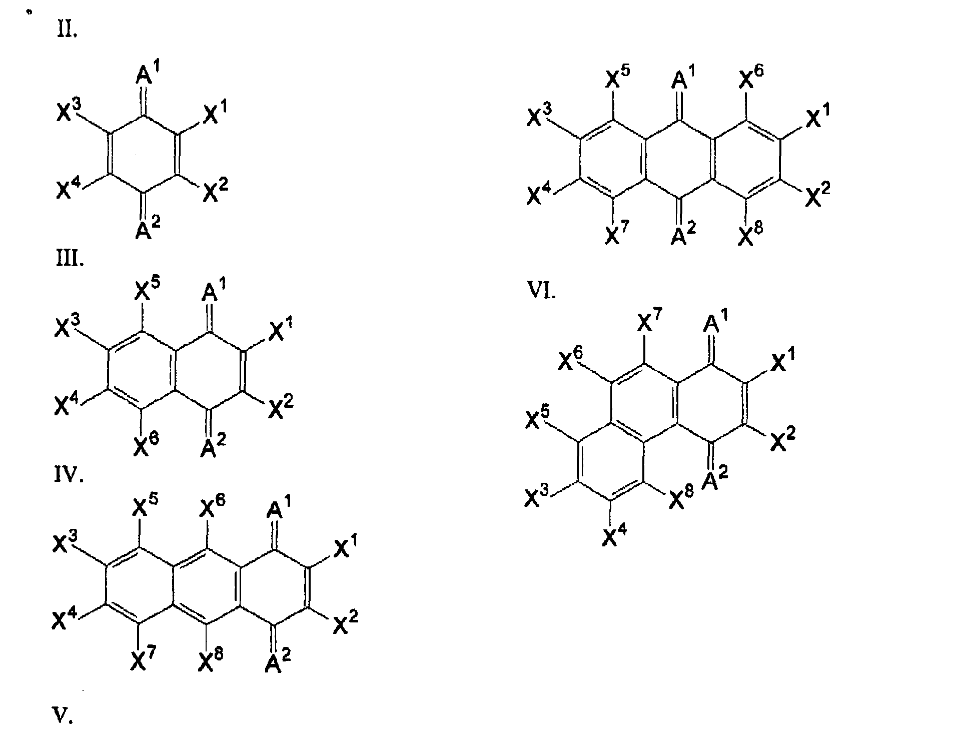 or

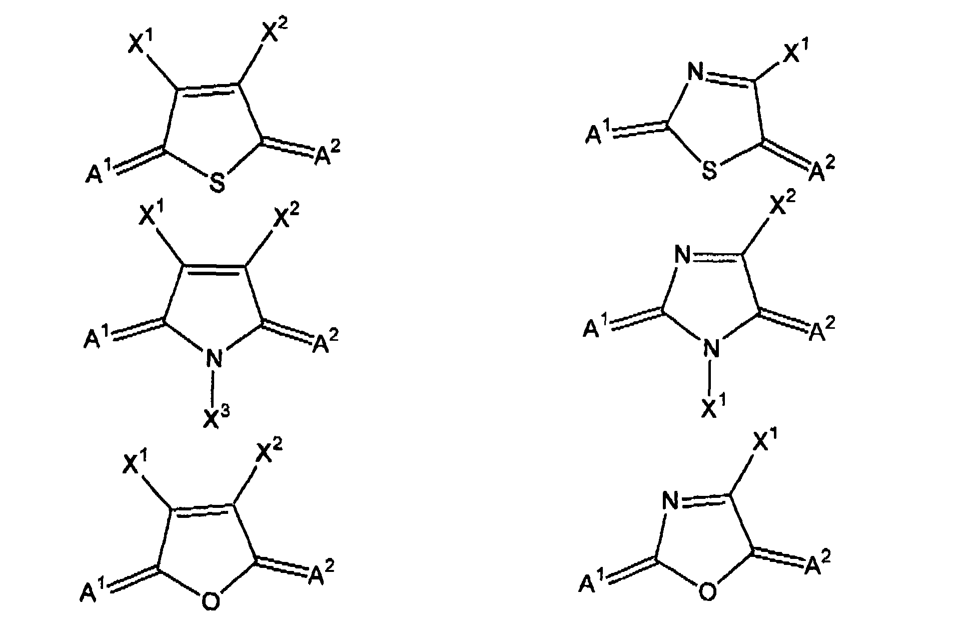

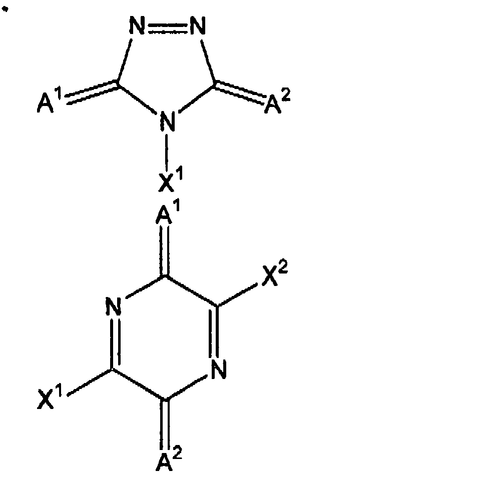

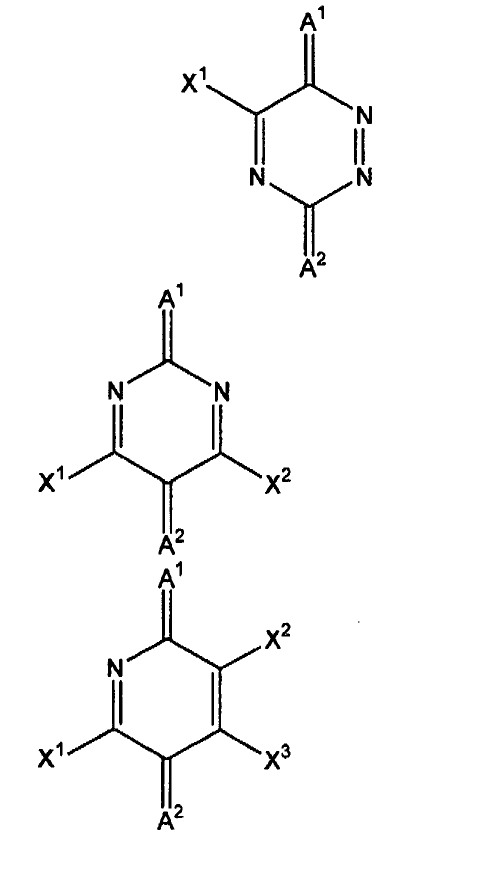

6. Utilisation selon une des revendications précédentes, **caractérisée par le fait que** 1 à 8 HTM sont compris, chaque HTM étant lié à l'un de X¹, X², X³, X⁴ à X⁸.

7. Utilisation selon une des revendications précédentes, **caractérisée par le fait que** X¹, X², X³, X⁴ à X⁸ qui sont substitués par HTM sont sélectionnés au sein du groupe comprenant -O-, -S-, -SiR¹R², -CR¹R², -CR¹=CR², -NR¹, -N=CR¹, -N=N-, et une liaison chimique.

8. Utilisation selon une des revendications précédentes, **caractérisée par le fait que** X¹, X² X³, X⁴ à X⁸ qui ne sont pas substitués par HTM sont sélectionnés à partir du groupe comprenant -H, -F, -CN, -OR¹, -SR¹, -NR¹R², -SiR¹R²R³, -CR¹R²R³, - CR¹=CR²R³, -N=NR¹, et HTM.

9. Utilisation selon une des revendications précédentes, **caractérisée par le fait que** HTM est sélectionné à partir du groupe comprenant des tris-[(*N*,*N*-diaryl)amino]-triphénylamines, des 4,4',4"-tfis[(*N*-(1-naphthyl)-*N*-phényl-amino-triphenylamine] (1-TNATA) et ses dérivés, des 4,4',4"-tris[(*N*-(2-naphthyl)-*N*-phényl amino)-triphénylamine] (2-TNATA) ; des 4,4',4"-tris((*N*-(3-méthylphényl)-*N-*phényl-amino)-triphénylamine] (m-TDATA) et ses dérivés, des 4,4',4"-tris(carbazole-9-yl)triphénylamines ; des *N*,*N*,*N*',*N*'-tétra arylbenzidines en tant que *N*,*N*,*N*',*N*'-tétra phényl benzidine et ses dérivés, des *N*,*N*'-bis(1-naphthyl)-*N*,*N*'-diphényl-benzidine (α-NPD), *N*,*N*'-di(naphthalène-2-yl)-*N*,*N*'-diphényl-benzidine (β-NPD), des 4,4'-bis(carbazole-9-yl)biphényl (CBP) et ses dérivés ; des 4,4'-bis(2,2'-diphénylvinyl)-1,1'-biphényl (DPVBI) ; des triarylamines et leur dérivés, des 4,4'-bis(*N*,*N-*diarylamino)-terphényls, des 4,4'-bis(*N*,*N*-diarylamino)-quarterphényls et leurs homologues et dérivés.

10. Utilisation selon la revendication 6, **caractérisée par le fait que** the HTM est un hétéroatome substitué de façon analogue à cela.

11. Utilisation selon la revendication 6, **caractérisée par le fait que** l'hétéroatome substitué de façon analogue est un dérivé thiényl-, sélényl- ou furanyl-.

12. Utilisation selon une des revendications précédentes, **caractérisée par le fait que** A¹ et A² sont sélectionnés de façon indépendante à partir du groupe comprenant les groupes cyano, -C(CN)₂, et -NCN.

13. Utilisation selon une des revendications précédentes, **caractérisée par le fait que** le composé est le 2,5-bis{4-[(4'-diphénylamino-biphényl-4-yl)-phénylamino]-benzyl)-1,4-bis-(dicyanométhylidene)-cyclohexa-2,5-diène **(14).**

14. Utilisation selon une des revendications précédentes, **caractérisée par le fait que** le composé est le 2,5-bis-(4-diphénylaminobenzyl)-1,4-bis-(dicyanométhylidène)-cyclohexa-2,5-diène **(19).**

15. Utilisation selon une des revendications précédentes en tant que couche de transport de trous ou d'électrons et/ou en tant que couche d'injection de trous ou d'électrons sans ajout d'autres matériaux de matrice.

16. Process destiné à synthétiser un composé pour l'utilisation selon une des revendications précédentes, **caractérisée par** les étapes de
a) réaction d'un groupe 1,4-cyclohexanédione di-substituée à une dicétal stable,
b) formation d'une liaison chimique non-conjuguée entre le groupe 1,4-cyclohexanédione avec au moins un HTM,
c) remplacement chimique des groupes cétone par des groupes donnant des électrons pour former une liaison conjuguée entre les groupes donnant des électrons et le groupe cyclohexyl, et
d) oxidation du 1,4-bis(dicyanométhylidéne)-cyclohexane en 1,4-bis(dicyano-méthylidéne)-1,4-cyclohexa-2,5-diène.

17. Dispositif électrique qui est un dispositif OLED, OFET, un dispositif laser ou un dispositif photovoltaïque, **caractérisé par** l'utilisation d'un composé selon une des revendications 1 à 16.

18. Process destiné à produire des dispositifs électriques ou électro-optiques, **caractérisé par le fait qu'**il comprend un composé selon une des revendications 1 à 15.

19. Process selon la revendication 18, **caractérisé par le fait que** les couches organiques du dispositif et l'électrode de contact final sont formés dans un process sous vide.

20. Process selon la revendication 19, **caractérisé par le fait que** le process sous vide est un PVD (déposition de vapeur physique), CVD (déposition de vapeur chimique) ou un process OVPD (déposition de vapeur organique physique).

21. Process selon la revendication 18, **caractérisé par le fait que** le composé utilisé selon une des revendications 1 à 15 est appliquée par revêtement à partir d'une solution ou d'un grésillement.

22. Process selon la revendication 21, **caractérisé par le fait que** le revêtement est un revêtement par pulvérisation, spin, immersion ou couteau.
